Europäisches Patentamt

European Patent Office

Office européen des brevets

(11) Numéro de publication: **0 320 331**
**A1**

## (12) DEMANDE DE BREVET EUROPEEN

(21) Numéro de dépôt: **88402963.8**

(22) Date de dépôt: **24.11.88**

(51) Int. Cl.⁴: **A 61 B 8/12**

(30) Priorité: **01.12.87 FR 8716634**

(43) Date de publication de la demande:
**14.06.89 Bulletin 89/24**

(84) Etats contractants désignés: **DE GB NL**

(71) Demandeur: **GENERAL ELECTRIC CGR SA.**
**13, Square Max-Hymans**
**F-75015 Paris (FR)**

(72) Inventeur: **Souquet, Jacques**
**Cabinet Ballot-Schmit 84 avenue Kléber**
**F-75116 Paris (FR)**

**Bele, Robert**
**Cabinet Ballot-Schmit 84 avenue Kléber**
**F-75116 Paris (FR)**

(74) Mandataire: **Ballot, Paul Denis Jacques et al**
**Cabinet Ballot-Schmit 84, avenue Kléber**
**F-75116 Paris (FR)**

(54) Sonde et appareil d'échographie.

(57) L'invention a principalement pour objet une sonde et un appareil d'échographie.

L'invention concerne une sonde d'échographie, par exemple rectale, comportant une barrette de transducteurs piézo-électriques (2) pouvant se déplacer. Avantageusement, la rotation de la barrette (14) de transducteurs piézo-électriques (2) permet d'amener la barrette, notamment, dans deux positions perpendiculaires (14A et 14B). Cela permet d'effectuer des prises de vues quand les plans de balayage sont perpendiculaires.

L'invention s'applique notamment à la réalisation des sondes d'échographie.

L'invention s'applique principalement à la réalisation des sondes médicales comme des sondes rectales.

FIG.2

EP 0 320 331 A1

**Description**

## SONDE ET APPAREIL D'ECHOGRAPHIE

L'invention a principalement pour objet une sonde et un appareil d'échographie. L'invention a notamment pour objet une sonde rectale d'échographie à ultrasons. Il est connu en échographie par ultrasons d'utiliser des barrettes de transducteurs piézo-électriques. Selon l'orientation d'une telle barrette on obtient des images différentes, par exemple de l'organe à visualiser.

Il est très important, notamment en échograhie rectale de pouvoir obtenir des images correspondants à deux positions perpendiculaires de barrettes de transducteurs piézo-électriques. Ainsi, on connaît une sonde rectale comportant deux barrettes distinctes orientées à 90°. Cette disposition rend très difficile la superposition et/ou la corrélation d'images obtenues par les deux barrettes dans la mesure où il existe une distance entre les deux barrettes. De plus, une telle construction nécessite l'emploi de deux barrettes de transducteurs piézoélectriques qui sont une pièce très coûteuse et dont la reproductibilité, surtout pour de très hautes résolutions, est difficile. Ainsi, il est possible que les deux barrettes n'aient pas de réponses identiques.

Dans une première variante de réalisation, on utilise une barrette linéaire.

Dans une seconde variante de réalisation, on utilise une barrette convexe.

La sonde selon la présente invention comporte au moins une barrette de transducteurs piézo-électriques susceptibles de se déplacer et ainsi de fournir des images pour les diverses inclinaisons de la barrette de transducteurs piézo-électriques. Avantageusement, la barrette est susceptible de tourner. La sonde selon l'invention présente des avantages importants par rapport à la sonde connue.

Un premier avantage provient de l'utilisation de la même barrette pour toutes les images. Il s'ensuit que la sensibilité, la directivité et la résolution de la barrette pour obtenir toutes les images est la même. De plus, la distance entre les deux positions de la barrette est faible. Ainsi, il est plus facile de corréler les images obtenues pour les diverses positions de la barrette par exemple en le superposant à l'écran ou en faisant un traitement numérique de l'image.

Un deuxième avantage de la disposition selon la présente invention est de n'avoir besoin que d'une seule barrette pour réaliser la sonde.

Un troisième avantage de la solution selon la présente invention est de permettre de réaliser les images correspondant aux positions intermédiaires de la barrette, par exemple à 1,10,30,40,45,50,60,80 et 90°.

L'invention sera mieux comprise au moyen de la description ci-après et des figures annexées données comme des exemples non limitatifs parmi lesquelles :

- la figure 1 est un schéma d'une sonde de type connu ;
- la figure 2 est un schéma d'un premier exemple de réalisation d'une sonde selon la présente invention ;

- la figure 3 est un schéma d'un deuxième exemple de réalisation d'une sonde selon la présente invention ;
- la figure 4 est un schéma d'un troisième exemple de réalisation d'une sonde selon la présente invention ;
- la figure 5 est un schéma d'un quatrième exemple de réalisation d'une sonde selon la présente invention ;
- la figure 6 est un schéma d'un détail de réalisation de la sonde selon la présente invention.

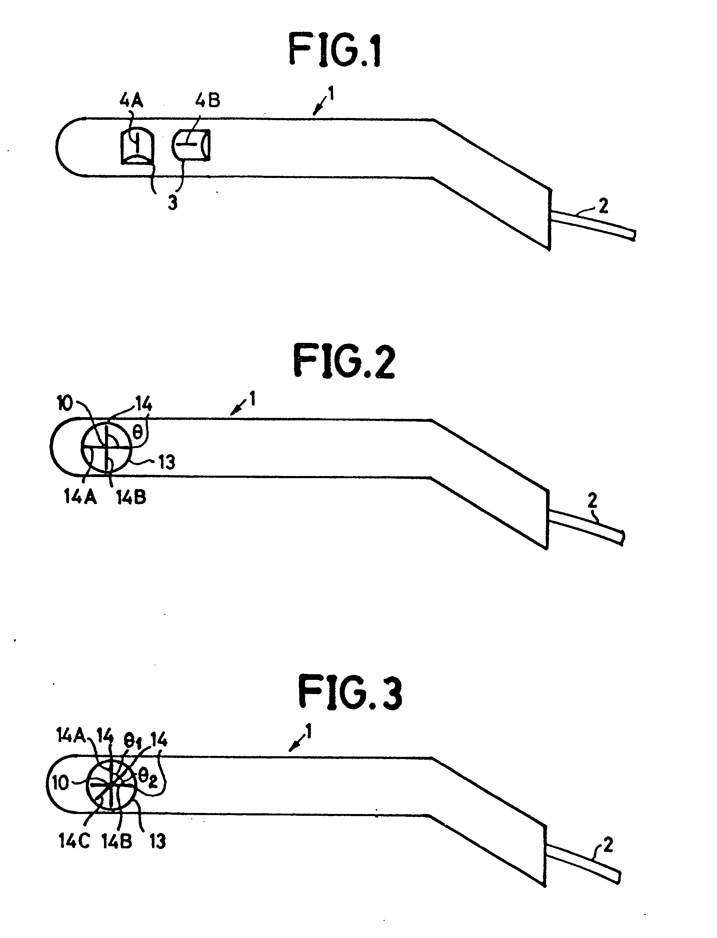

Sur les figures 1 à 6 on a utilisé les mêmes références pour désigner les mêmes éléments.

Sur la figure 1, on peut voir une sonde rectale 1 de type connu. La sonde 1 comporte deux zones distinctes d'émission-réception 3, chaque zone comportant une barrette respectivement 4A et 4B de transducteurs piézo-électriques. La barrette 4A est disposée à 90° par rapport à la barrette 4B. La sonde 1 est reliée par un câble 2 à un dispositif d'imagerie par ultrasons non représenté.

L'utilisation des deux barrettes 4A et 4B permet d'effectuer un balayage dans deux plans orthogonaux. Ainsi, il est possible de choisir l'image la plus intéressante, par exemple pour effectuer un diagnostic. Toutefois, il existe un décalage entre les zones d'émission-réception 3. Ce décalage rend difficile la corrélation ou la comparaison des images obtenues par l'intermédiaire des barrettes 4A et 4B. Le dispositif de type connu présente, de plus, l'inconvénient de nécessiter une barrette par position. Ainsi, avec les dispositifs de type connu, et vu le prix des barrettes, seules deux orientations des barrettes 4A ou 4B sont accessibles.

Sur la figure 2, on peut voir un premier exemple de réalisation de sonde d'échographie selon la présente invention. La sonde d'échographie rectale 1 comporte une zone 13 comportant une barrette linéaire 14. La zone d'émission-réception 13 est mobile de façon à permettre à la barrette 14 de prendre plusieurs positions et notamment de prendre des positions présentant un angle , par exemple égal, à 90°. Les exemples de positions extrêmes sont référencées 14A et 14B sur la figure 2. La barrette est susceptible d'être figée dans les positions 14A et 14B.

Sur la figure 2, on voit la barrette 14 dans deux positions distinctes 14A et 14B. Il est bien entendu que le dispositif d'émission-réception 13 ne comporte qu'une seule barrette 14.

Dans l'exemple de la figure 2, le déplacement de la position 14B à la position 14B est obtenue par une rotation autour d'un axe passant par le milieu de la barrette 14. Il est bien entendu que d'autres types de déplacement de la zone 13 entraînant une modification de l'orientation de la barrette 14 ne sort pas du cadre de la présente invention.

Dans une première variante de réalisation du dispositif selon la présente invention le déplacement de la position 14A à la position 14b est obtenu manuellement, la sonde étant à l'air libre.

Avantageusement, la sonde 1 comporte des moyens électromécaniques permettant de déplacer la zone 13. Ces moyens électromécaniques sont par exemple un moteur électrique ou un électro-aimant. Avantageusement, la zone 13 présente une surface lisse permettant sa rotation à l'intérieur même du patient. Ainsi, il est possible d'effectuer des vues d'une même portion du patient sous divers angles de la barrette 14.

Il est bien entendu que les barrettes 14 pouvant prendre n'importe quel angle θ ne sortent pas du cadre de la présente invention.

Un câble 2 relie la sonde 1 selon la présente invention à un appareil d'imagerie par ultrasons. Ce câble permet la transmission des données recueillies par la sonde, l'alimentation électrique de la sonde, l'alimentation électrique ainsi que les signaux de commande de la zone 13.

Sur la figure 3, on peut voir une sonde 1 avec la barrette 14 comporte une position de repos 14C. La barrette 14 amenée en position 14A et 14B par une rotation d'angle $+ \theta 1$ ou $- \theta 2$. Cela permet de limiter le débattement du mouvement de la barrette 14 et par là même les connexions notamment des transducteurs piézo-électriques. Avantageusement $\theta 1 = \theta 2 = 45°$.

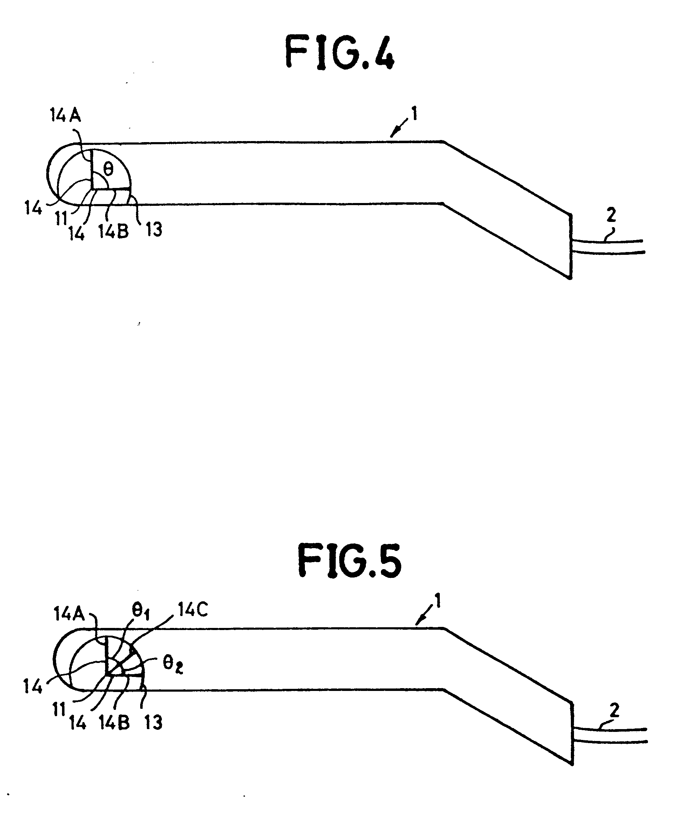

Sur la figure 4, on peut voir un exemple de réalisation de la sonde 1 selon la présente invention dans lequel la barrette 14 est susceptible de tourner autour d'un axe passant par l'une de ses extrémités. Cette variante de réalisation présente l'inconvénient de nécessiter une zone 13 plus étendue.

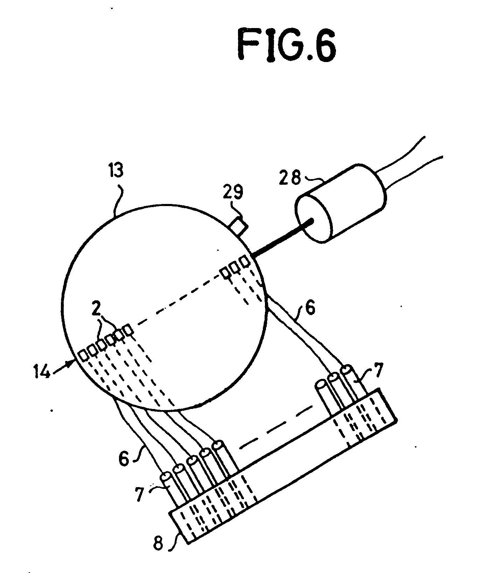

Sur la figure 5, on peut voir une variante de réalisation d'une sonde selon la présente invention. La barrette 14 de la sonde 1 de la figure 5 est susceptible de tourner autour d'un axe passant par l'une de ses extrémités. La barrette 14 comprend une position de repos référencée 14C, les positions extrêmes 14A et 14B sont atteintes par une rotation de $\theta 1$ respectivement $- \theta 2$.

Sur la figure 6, on peut voir les connexions des transducteurs piézo-électriques 2 des barrettes 14 des figures 2,3. Chaque transducteur est relié par un câble coaxial comportant une âme 6 et un blindage 7. Avantageusement, le blindage 7 constitue la masse. Avantageusement, tous les blindages 7 sont reliés par exemple à une barrette métallique 8. La rotation de la zone 13 est permise par la souplesse des fils 6, l'alimentation des transducteurs 2.

Le moteur d'entraînement de la zone 13 porte la référence 28.

Les fils coaxiaux ainsi que les fils d'alimentation et de commande du moteur 28 passent par le câble 2. Ainsi, il est possible à partir d'un appareil d'imagerie ultrasonore selon l'invention de télécommander la position de la barrette 14. Le système de commande est adapté au moteur 28 utilisé. Par exemple, on peut utiliser un interrupteur à au moins deux positions, une résistance variable de type potentiomètre. Si on utilise un moteur 28 pas à pas la commande de position comporte par exemple deux boutons susceptibles d'envoyer des impulsions permettant de faire tourner dans un sens ou dans l'autre le moteur 28. Avantageusement, la zone 13 comporte une butée 29 empêchant un débattement trop grand de la zone 13 qui risquerait d'endommager les fils 6. Une ou deux butées correspondantes se trouvent sur la sonde 1.

L'invention s'applique notamment à la réalisation des sondes d'échographie.

L'invention s'applique principalement à la réalisation des sondes médicales comme des sondes rectales.

**Revendications**

1. Sonde endo-rectale (1) d'échographie comportant une barrette linéaire ou convexe (4A,4B,14) de transducteurs (2) piézo-électriques, caractérisée par le fait que la barrette de transducteurs (2) piézo-électriques est mobile autour d'un axe perpendiculaire à l'axe longitudinal de la sonde.

2. Sonde selon la revendication 1, caractérisée par le fait que la barrette est susceptible d'être figée dans au moins deux positions.

3. Sonde selon la revendication 2, caractérisée par le fait que l'axe de rotation est situé au centre de la barrette.

4. Sonde selon la revendication 2, caractérisée par le fait que l'axe de rotation est situé à l'une des extrémités de la barrette.

5. Sonde selon la revendication 1,2,3 ou 4, caractérisée par le fait que la barrette (14) a un débattement de $\pm 45°$ autour d'une position centrale.

6. Sonde selon la revendication 1,2,3,4 ou 5, caractérisée par le fait qu'elle comporte un moteur susceptible d'entraîner la barrette (14) de transducteurs piézo-électriques (2).

7. Sonde selon la revendication 1,2,3,4,5 ou 6, caractérisée par le fait que la barrette (14) est une barrette linéaire.

8. Sonde selon la revendication 1,2,3,4,5 ou 6, caractérisée par le fait que la barrette (14) est une barrette convexe.

9. Appareil d'échographie caractérisé par le fait qu'il comporte une sonde selon l'une quelconque des revendications précédentes.

10. Appareil selon la revendication 8, caractérisé par le fait qu'il comporte des moyens de télécommande de la position de la barrette (14) de transducteurs (2) piézo-électriques.

# FIG.1

# FIG.2

# FIG.3

# FIG.4

# FIG.5

# FIG.6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 139 574 (B.D.L. FORNAGE)<br>* Page 3, lignes 1 - 34; page 8, ligne 26 - page 9, ligne 16; figures 1,2,7,8,9 *<br>--- | 1,7,8 | A 61 B    8/12 |
| A | EP-A-0 039 045 (OLYMPUS OPTICAL CO LTD)<br>* Page 2, ligne 34 - page 4, ligne 22; figures 1 - 3 *<br>--- | 1,6,7, 10 | |
| A | EP-A-0 065 275 (OLYMPUS OPTICAL CO LTD)<br>* Résumé; figure 2 *<br>----- | 1,6 | |

DOMAINES TECHNIQUES
RECHERCHES (Int. Cl.4)

A 61 B    8/00
A 61 B    10/00

Le présent rapport a été établi pour toutes les revendications

| Lieu de la recherche | Date d'achèvement de la recherche | Examinateur |
|---|---|---|
| BERLIN | 16-02-1989 | WEIHS J.A. |